# FASCICULE DE BREVET EUROPEEN

(11) **EP 2 875 841 B1**
(45) Date de publication et mention de la délivrance du brevet: **13.01.2021**
(21) Numéro de dépôt: 14194367.0
(22) Date de dépôt: 21.11.2014
(51) Int. Cl.: A61M 25/00, A61M 16/04, A61M 25/01

(54) **Sonde pour injecter un agent fluide chez un nouveau-né ou un prématuré sous ventilation non-invasive en pression positive continue**
Sonde zum Injizieren einer flüssigen Substanz bei einem Neugeborenen oder Frühchen unter nicht-invasiver Beatmung mit kontinuierlichem positiven Druck
Probe for injecting a fluid agent into a newborn or premature receiving non-invasive ventilation with continuous positive pressure

(30) Priorité: 21.11.2013 FR 1361490
(43) Date de publication de la demande: 27.05.2015
(73) Titulaire: Vygon, 95440 Ecouen (FR)
(72) Inventeur: Bertheuil, Elodie, 95430 Auvers sur Oise (FR); Letourmy, Christelle, 60280 Margny-les-Compiegne (FR); Kribs, Angela, 50931 Cologne (DE)
(74) Mandataire: Regimbeau

(56) Documents cités:
- WO-A1-2013/166471
- WO-A1-2014/113821
- US-A- 5 509 408
- US-A- 5 954 636
- US-A1- 2005 103 332
- US-A1- 2008 262 428
- US-A1- 2012 298 112

## Description

### DOMAINE DE L'INVENTION

L'invention concerne notamment l'injection d'un ou plusieurs agents fluides dans le cadre d'une ventilation non-invasive d'une personne afin de prévenir entre autres le collabage de ses alvéoles pulmonaires. Plus précisément, l'invention concerne l'injection de surfactant dans les alvéoles pulmonaire d'un nouveau-né ou d'un prématuré sous ventilation en pression positive continue non-invasive.

### ARRIERE-PLAN TECHNOLOGIQUE

L'injection de surfactant dans le cadre de la réanimation du prématuré a montré son efficacité pour prévenir le collabage de ses alvéoles pulmonaires. Elle est de plus en plus utilisée dans les services hospitaliers, aussi bien lorsque le prématuré est sous ventilation invasive (sonde endotrachéale) ou non-invasive avec un masque par CPAP (acronyme anglais de Continuous Positive Airway Pressure, pour ventilation en pression positive continue).

Dans le cas de la ventilation invasive, les enfants sont intubés à l'aide d'une sonde endotrachéale à un ou à deux canaux, avec un canal primaire adapté pour ventiler l'enfant et un canal secondaire adapté pour injecter du surfactant une fois que la sonde est positionnée dans la trachée de l'enfant. L'injection est facile et rapide. Néanmoins, l'intubation des enfants est dangereuse et peut provoquer chez eux des dommages, notamment au niveau de leur trachée, de leur larynx ou encore des tissus environnants.

A l'heure actuelle, la préférence est donc donnée à la ventilation non invasive à l'aide d'un masque équipé d'une CPAP par voie orale ou nasale. Il reste cependant nécessaire d'injecter du surfactant au cours de la ventilation afin de décoller les alvéoles des poumons et permettre les échanges gazeux avec le sang.

La méthode couramment utilisée en salle d'accouchement consiste à ventiler sous CPAP l'enfant par le biais d'un masque (oral ou nasal), puis d'introduire :
- Soit : une sonde endotrachéale à l'aide d'un laryngoscope dans sa trachée. On peut alors injecter le surfactant à l'aide de la sonde endotrachéale, puis la retirer.
- Soit une sonde gastrique ou une sonde d'aspiration à l'aide d'une pince.

Toutefois, ces méthodes présentent plusieurs inconvénients majeurs.

Tout d'abord, l'introduction de la sonde endotrachéale risque d'irriter la trachée de l'enfant, en particulier lorsqu'il s'agit d'un nouveau-né ou d'un prématuré, ce qui requière un suivi médical très important. De plus, la sonde endotrachéale, qui appuie sur la trachée, déclenche par réflexe l'arrêt des mouvements du diaphragme de l'enfant, qui ne respire donc plus spontanément. Or des études récentes ont montré que le surfactant injecté lorsque l'enfant est sous respiration spontanée a une meilleure distribution dans les alvéoles et est donc plus efficace.

Ensuite, l'utilisation d'une sonde gastrique ou d'aspiration n'est pas adaptée à l'injection de surfactant, et le retrait de la pince, qui est requis avant l'injection du surfactant, peut déplacer le positionnement de l'extrémité distale. Ceci peut entrainer une moins bonne distribution du surfactant et allonger le temps de l'intervention.

Les documents US 2005/103332, US 2008/0262428 et US 5 509 408 ont donc proposé une sonde comprenant une partie proximale comprenant une extrémité proximale munie d'un raccord proximal, et une partie fixée solidairement à l'extrémité distale de la partie proximale réalisée dans un matériau plus souple que la partie proximale. Le document US 5 509 408 décrit une sonde selon le préambule de la revendication 1.

Toutefois, ces sondes ne sont pas adaptées pour des nouveau-nés ou des prématurés et risquent de l'irriter lors de leur insertion ou de leur extraction dans la trachée et/ou de gêner sa ventilation.

### RESUME DE L'INVENTION

Un objectif de l'invention est donc de proposer un dispositif adapté permettant d'injecter facilement et rapidement un ou plusieurs agents fluides dans la trachée d'une personne, et plus particulièrement d'un enfant (nouveau-né ou prématuré), alors que cette personne est ventilée de manière non-invasive, et ce sans perturber sa respiration spontanée.

Pour cela, l'invention propose une sonde d'injection d'un surfactant selon la revendication 1.

Certaines caractéristiques préférées mais non limitatives de la sonde sont les suivantes :
- la partie proximale est formée dans un matériau semi-rigide présentant une dureté comprise entre 60 Shore D et 100 Shore D, de préférence entre 65 Shore D et 80 Shore D, par exemple 72 Shore D,
- la partie distale est formée dans un matériau souple présentant une dureté comprise entre 20 Shore D et 60 Shore D, de préférence entre 35 Shore D et 55 Shore D,
- le matériau de la partie distale (3) et/ou la partie proximale (2) est déformable plastiquement à froid à la main,
- la partie proximale comprend en outre une lumière, adaptée pour recevoir une tige de rigidification configurée pour rigidifier ladite partie proximale, par exemple une tige métallique,
- la tige de rigidification est coextrudée avec la lumière ou insérée dans ladite lumière,
- la partie distale comprend un matériau élastomère thermoplastique du type polyéther block amide (PEBA),
- la partie distale forme un angle α compris entre 40° et 45° avec la partie proximale,
- la partie proximale et la partie distale sont courbes,
- la partie proximale et/ou la partie distale comprend un marquage,
- la partie distale et la partie proximale sont de couleur distincte,
- la partie distale mesure entre 0.5 cm et 4.0 cm de long, et la partie proximale mesure entre 15 cm et 25 cm de long.

### BREVE DESCRIPTION DES DESSINS

D'autres caractéristiques, buts et avantages de la présente invention apparaîtront mieux à la lecture de la description détaillée qui va suivre, et au regard des dessins annexés donné à titre d'exemples non limitatifs et sur lesquels :
La figure 1 est un premier exemple de réalisation d'une sonde conforme à un mode de réalisation de l'invention, et

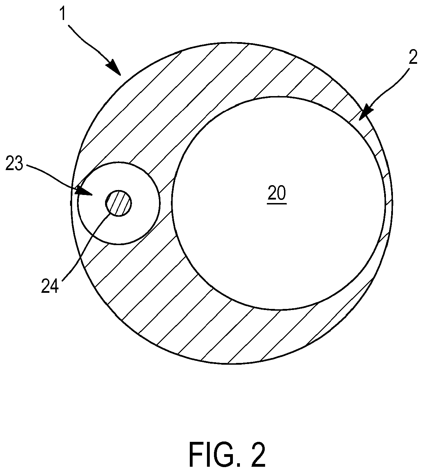

La figure 2 est une vue en coupe transversale d'une partie proximale d'un deuxième exemple de réalisation d'une sonde conforme à un mode de réalisation de l'invention.

### DESCRIPTION DETAILLEE D'UN MODE DE REALISATION

L'invention propose entre autres un dispositif pour l'injection dans un conduit d'une personne d'un ou plusieurs agents fluides tout en permettant sa ventilation non-invasive. Dans ce qui suit, l'invention va plus particulièrement être décrite dans le cas de l'injection d'un agent fluide du type surfactant dans les alvéoles pulmonaires d'un enfant, de préférence un nouveau-né ou un prématuré, à travers sa trachée. Ceci n'est cependant pas limitatif, dans la mesure où le dispositif peut être utilisé pour injecter d'autres types d'agents fluides, être introduit dans d'autres conduits, et/ou être utilisé pour des adultes.

Par ailleurs, dans la suite de description, « proximal » désignera une pièce (par exemple une partie) qui se situe à proximité de l'opérateur lorsque celui-ci utilise le dispositif de l'invention, tandis que « distal » désignera une pièce qui est éloignée de l'opérateur au cours de cette utilisation.

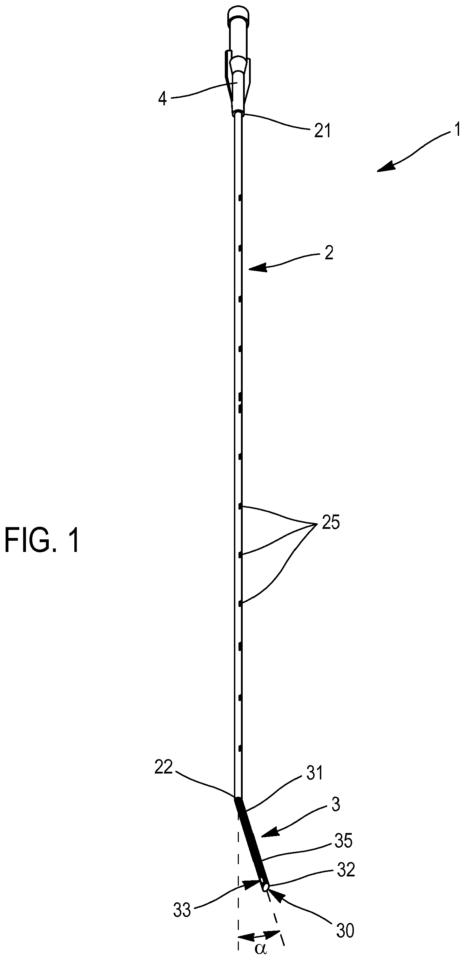

Un exemple d'un tel dispositif est illustré en figure 1.

Le dispositif peut être une sonde 1, comprenant une partie proximale 2 et une partie distale 3.

La partie proximale 2 définit un passage proximal 20 et comprend une extrémité proximale 21 munie d'un raccord proximal 4 et une extrémité distale 22, opposée à l'extrémité proximale 21. Le raccord proximal 4 est en communication fluidique avec le passage proximal 20, et est configuré pour permettre la connexion d'un moyen d'injection du surfactant à travers la sonde 1. Par exemple, le raccord proximal 4 peut être de type Luer, Luer Lock ou tout raccord conforme aux normes en vigueur, et peut être configuré pour coopérer avec l'embout d'une seringue comprenant le surfactant.

La partie distale 3 quant à elle défini un passage distal 30 en communication fluidique avec le passage proximal 20 de la partie proximale 2. La partie distale 3 comprend une extrémité proximale 31, fixée solidairement à l'extrémité distale 22 de la partie proximale 2, et une extrémité distale 32 débouchante afin de permettre l'injection du surfactant dans les alvéoles pulmonaires de l'enfant. Optionnellement, un orifice 33 débouchant dans le passage distal 30 peut en outre être formé, dans une zone adjacente à l'extrémité distale 32. Cet orifice 33 peut notamment s'étendre latéralement par rapport à la direction d'extension de la partie distale 3, et permet de garantir le passage de l'agent fluide, même lorsque l'extrémité distale 32 est obstruée.

La partie proximale 2 et la partie distale 3 peuvent être formées intégralement et en une seule pièce, par exemple par coextrusion. En variante, la partie distale 3 peut être rapportée et fixée par collage ou soudage sur l'extrémité distale de la partie proximale 2.

La sonde 1 peut mesurer une vingtaine de centimètres de long. La partie distale 3 peut par exemple mesurer entre 0.5 cm et 4.0 cm de long, de préférence de l'ordre de 2.0 cm de long, tandis que la partie proximale 2 peut mesurer entre 15 cm et 25 cm de long.

Par ailleurs, la sonde 1 peut présenter un diamètre externe sensiblement constant compris entre environ 4/3 mm (4 Fr) et 2.0 mm (6 Fr), et un diamètre interne sensiblement constant compris entre environ entre 0.5 mm et 1.2 mm. Par exemple, le diamètre interne et le diamètre externe de la sonde 1 peuvent être constants sur toute sa longueur, soit de sa partie proximale 2 à sa partie distale 3, et être égal à 1.2*2.0 mm ou encore 0.8*2.0 mm. De la sorte, l'opérateur peut introduire la sonde 1 dans la trachée sans déclencher par réflexe l'arrêt des mouvements du diaphragme de l'enfant. En effet, contrairement aux sondes endotrachéale habituellement utilisées, la sonde 1 de l'invention ne prend pas appui sur sa trachée grâce à son faible diamètre. L'enfant peut donc continuer à respirer spontanément, ce qui améliore la distribution et l'efficacité du surfactant par la sonde 1 dans les alvéoles pulmonaires.

De préférence, la partie distale 3 est réalisée dans un matériau plus souple que la partie proximale 2 afin d'éviter tout endommagement lors de son introduction dans la trachée, tout en permettant à l'opérateur de guider avec précision la sonde 1 jusqu'aux cordes vocales à l'aide d'un laryngoscope (ou tout autre instrument adapté).

Ainsi, la partie proximale 2 peut par exemple être réalisée dans un matériau semi-rigide, afin de permettre le guidage par un opérateur de la sonde 1 lors de son introduction dans la trachée. La Demanderesse s'est en effet aperçue que l'utilisation d'un matériau trop souple pour la partie proximale 2 rendait difficile la manipulation et le guidage de la sonde 1 par l'opérateur. Cela augmente en outre le temps de l'injection, l'inconfort du bébé et risque d'irriter la trachée en cas de mauvaise manipulation.

Par exemple, le matériau semi-rigide de la partie proximale 2 peut présenter une dureté comprise entre 60 Shore D et 100 Shore D, de préférence entre 65 Shore D et 80 Shore D, par exemple 72 Shore D. Ainsi, dans une forme de réalisation, le matériau semi-rigide de la partie proximale 2 comprend du polyéther block amide (PEBA) ayant une dureté de 72 Shores D.

La partie distale 3 quant à elle peut être réalisée dans un matériau plus souple que la partie proximale 2, afin d'éviter tout traumatisme ou irritation de la muqueuse de l'enfant lors de l'introduction de la sonde 1 dans sa trachée. Par exemple, le matériau plus souple de la partie distale 3 peut présenter une dureté comprise entre 20 Shore D et 60 Shore D, de préférence entre 35 Shore D et 55 Shore D. Ainsi, dans une forme de réalisation, le matériau plus souple de la partie distale 3 comprend du polyéther block amide (PEBA) ayant une dureté de 35 ou 55 Shore D.

La partie proximale 2 et la partie distale 3 sont de forme globalement allongée. Par exemple, la partie proximale 2 et la partie distale 3 peuvent être de forme sensiblement cylindrique, voire cylindriques de révolution.

Selon l'invention, illustrée sur la figure 1, la partie distale 3 est inclinée par rapport à la partie proximale 2 selon un angle α compris entre 30° et 60°, de préférence entre 40° et 45°, et former une sorte de béquille permettant de simplifier l'insertion de la sonde 1 dans la trachée.

Cette forme de réalisation est particulièrement adaptée dans le cas d'une insertion orale de la sonde 1.

Avantageusement, grâce à la forme inclinée de la partie distale 3 de la sonde 1, il n'est donc pas nécessaire d'utiliser d'instrument spécifique pour introduire la sonde 1 dans la trachée, hormis un laryngoscope (ou équivalent). Il n'y a donc pas de risque de traumatisme de la trachée due à une mauvaise manipulation de l'instrument, ni de délogement de la sonde 1 lors du retrait dudit instrument.

L'inclinaison de la partie distale 3 par rapport à la partie proximale 2 peut être réalisée lors de la fabrication de la sonde 1.

En variante, la partie distale 3 peut être réalisée dans un matériau susceptible d'être déformé à froid manuellement par l'opérateur et être capable de conserver cette déformation pour son introduction dans la trachée (déformation plastique). Le cas échéant, la partie proximale 2 et la partie distale 3 peuvent être sensiblement rectilignes avant leur manipulation par l'opérateur. Dans cette variante de réalisation, l'opérateur peut alors préformer la partie distale 3 de la sonde 1 et lui conférer l'inclinaison souhaitée préalablement à son introduction dans la trachée.

Un exemple de matériau déformable pouvant être utilisé pour la partie distale 3 comprend un matériau élastomère thermoplastique du type polyéther block amide (PEBA).

Selon une deuxième forme de réalisation, la sonde 1 peut être incurvée sur toute sa longueur, c'est-à-dire de l'extrémité proximale 21 de la partie proximale 2 à l'extrémité distale 32 de la partie distale 3.

Cette forme de réalisation est particulièrement adaptée dans le cas d'une insertion nasale de la sonde 1.

La courbure de la sonde 1 peut notamment être obtenue lors de la fabrication de la sonde 1.

En variante, la sonde 1 peut être réalisée dans des matériaux (pour la partie proximale 2 et la partie distale 3) déformables à froid manuellement par l'opérateur et capables de conserver cette déformation pour son introduction dans la trachée (déformation plastique), tel que des matériaux élastomères thermoplastiques du type polyéther block amide (PEBA). L'opérateur peut alors préformer la sonde 1 et lui conférer la courbure souhaitée préalablement à son introduction dans la trachée.

Cette variante de réalisation présente l'avantage de rendre la sonde 1 utilisable à la fois par voie orale et par voie nasale. En effet, la sonde 1 peut être laissée rectiligne, ou éventuellement déformée uniquement au niveau de sa partie distale 3 afin de former la béquille, ou être incurvée sur toute sa longueur afin de faciliter son introduction par voie nasale.

Afin de rigidifier la partie proximale 2 de la sonde 1, celle-ci peut en outre comprendre une lumière 23, distincte du passage proximal 20, adaptée pour recevoir une tige de rigidification 24. La tige de rigidification 24 peut par exemple être une tige métallique.

De préférence, la tige de rigidification est déformable à froid manuellement par l'opérateur et capable de conserver cette déformation pour l'introduction de la sonde dans la trachée (déformation plastique). De la sorte, la tige de rigidification 24 permet de faciliter la déformation plastique de la sonde 1 afin de lui conférer la courbure souhaitée.

La tige de rigidification 24 peut être coextrudée avec la partie proximale 2. En variante, la partie proximale 2 peut être préalablement fabriquée de manière à former le passage proximal 20 et la lumière, puis la tige de rigidification 24 peut être rapportée dans ladite lumière 23.

La sonde 1 peut en outre comprendre des marqueurs 25, 35 formés sur sa partie proximale 2 et/ou sa partie distale 3.

Par exemple, la partie distale 3 peut être marquée en tout ou partie d'une couleur, notamment du noir, afin de simplifier son repérage par l'opérateur lors de son introduction par voie orale derrière les cordes vocales de l'enfant. Typiquement, le marquage 35 peut recouvrir toute la surface de la partie distale 3 de la sonde 1, ou du moins ses deux derniers centimètres.

Par ailleurs, la partie proximale 2 peut être marquée selon un intervalle défini, tel que tous les un ou deux centimètres, afin de simplifier son introduction par voie nasale.

La sonde 1 peut en outre être munie d'une fibre optique, débouchant au niveau de son extrémité distale 32, afin d'aider l'opérateur lors de l'introduction de la sonde 1. Le cas échéant, l'opérateur peut alors se passer de laryngoscope ou de tout autre instrument habituel pour introduire la sonde 1 dans la trachée de la personne.

La fibre optique peut alors être intégrée à la sonde 1, ou simplement glissée dans les passages proximal 20 et distal 30 (auquel cas elle est alors retirée de la sonde 1 préalablement à l'injection du surfactant).

Une telle sonde 1 peut être introduite dans la trachée d'une personne alors que celle-ci est sous ventilation non-invasive avec un masque pour CPAP orale ou nasale.

L'injection du ou des agents dans les alvéoles pulmonaires de l'enfant à l'aide d'une sonde 1 conforme à l'invention peut alors être réalisée conformément aux étapes suivantes.

Au cours d'une première étape, l'enfant est ventilé sous CPAP par le biais d'un masque oral ou nasal. Dans le cas d'un masque oral, celui-ci comprend de préférence un système ouvert permettant l'introduction de la sonde 1.

Au cours d'une deuxième étape, dans le cas d'un masque nasal où la bouche du patient est libre d'accès, une sonde 1 conforme à l'invention est alors introduite par voie orale dans la trachée de l'enfant, ou par voie nasale en libérant une narine du masque. L'introduction de la sonde 1 peut être réalisée à l'aide d'un laryngoscope et/ou d'une fibre optique, ou tout autre instrument adapté.

Dans le cas d'un masque oral, qui couvre à la fois le nez et la bouche du patient, l'introduction se fait par la bouche par le système ouvert du masque et de la CPAP formés au niveau de la bouche du patient. Dans les deux cas, l'extrémité distale 32 de la sonde 1 est amenée jusque derrière les cordes vocales de l'enfant.

La partie distale 3, plus souple, est facilement guidée grâce à la partie proximale 2 de la sonde 1, qui est semi-rigide, sans traumatiser l'enfant. Par ailleurs, les dimensions, et notamment le diamètre externe spécifique de la sonde 1, permettent à l'opérateur d'éviter de déclencher par réflexe l'arrêt des mouvements du diaphragme.

Au cours d'une troisième étape, l'opérateur peut alors injecter l'agent fluide dans les alvéoles pulmonaires de l'enfant, par exemple un surfactant. Pour cela, l'opérateur connecte au raccord proximal 4 une seringue remplie du surfactant, puis injecte à travers le passage proximal 20 et le passage distal 30 la quantité de surfactant désirée. Avec la même seringue, l'opérateur injecte ensuite de l'air pour pousser dans la trachée du patient le surfactant resté dans le passage proximal 20 et le passage distal 30.

On notera qu'au cours de la première et de la deuxième étape, la ventilation sous CPAP de l'enfant est maintenue à l'aide du masque et n'est en aucun cas perturbée par la présence de la sonde 1.

Enfin, au cours d'une quatrième étape, la sonde 1 peut être retirée.

## Revendications

1. Sonde (1) d'injection d'un surfactant dans une trachée d'une personne, notamment un nouveau-né ou un prématuré, comprenant :
- une partie proximale (2) définissant un passage proximal (20), ladite partie proximale (2) comprenant une extrémité proximale (21) munie d'un raccord proximal (4) et une extrémité distale (22), opposée à l'extrémité proximale (21), et
- une partie distale (3), définissant un passage distal (30) en communication fluidique avec le passage proximal (20), ladite partie distale (3) étant fixée solidairement à l'extrémité distale (22) de la partie proximale (2),
- la partie distale (3) étant réalisée
dans un matériau plus souple que la partie proximale (2) afin de faciliter son introduction dans le conduit et d'éviter tout endommagement dudit conduit, la sonde (1) étant **caractérisée en ce que** la partie proximale (2) présente un diamètre externe compris entre 4/3 mm et 2.0 mm (entre 4 Fr et 6 Fr), et **en ce que** la partie distale (3) forme un angle (α) compris entre 30° et 60° avec la partie proximale (2).

2. Sonde (1) selon la revendication 1, dans laquelle la partie proximale (2) est formée dans un matériau semi-rigide présentant une dureté comprise entre 60 Shore D et 100 Shore D, de préférence entre 65 Shore D et 80 Shore D, par exemple 72 Shore D.

3. Sonde (1) selon l'une des revendications 1 ou 2, dans laquelle la partie distale (3) est formée dans un matériau souple présentant une dureté comprise entre 20 Shore D et 60 Shore D, de préférence entre 35 Shore D et 55 Shore D.

4. Sonde (1) selon l'une des revendications 1 à 3, dans laquelle le matériau de la partie distale (3) et/ou la partie proximale (2) est déformable plastiquement à froid à la main.

5. Sonde (1) selon l'une des revendications 1 à 4, dans laquelle la partie proximale (2) comprend en outre une lumière (23), adaptée pour recevoir une tige de rigidification (24) configurée pour rigidifier ladite partie proximale (2), par exemple une tige métallique.

6. Sonde (1) selon la revendication 5, dans laquelle la tige de rigidification (24) est coextrudée avec la lumière (23) ou insérée dans ladite lumière (23).

7. Sonde (1) selon l'une des revendications 1 à 6, dans laquelle la partie distale (3) comprend un matériau élastomère thermoplastique du type polyéther block amide (PEBA).

8. Sonde (1) selon l'une des revendications 1 à 7, dans laquelle la partie distale (3) forme un angle (α) compris entre 40° et 45° avec la partie proximale (2).

9. Sonde (1) selon l'une des revendications 1 à 8, dans laquelle la partie proximale (2) et la partie distale (3) sont courbes.

10. Sonde (1) selon l'une des revendications 1 à 9, dans lequel la partie proximale (2) et/ou la partie distale (3) comprend un marquage (25, 35).

11. Sonde (1) selon la revendication 10, dans lequel la partie distale (3) et la partie proximale (2) sont de couleur distincte.

12. Sonde (1) selon l'une des revendications 1 à 11, dans laquelle la partie distale (3) mesure entre 0.5 cm et 4.0 cm de long, et la partie proximale (3) mesure entre 15 cm et 25 cm de long.

## Patentansprüche

1. Sonde (1) zum Injizieren eines Tensids in die Luftröhre einer Person, insbesondere eines Neugeborenen oder eines Frühchens, die umfasst:
- einen proximalen Teil (2), der einen proximalen Durchgang (20) definiert, wobei der proximale Teil (2) ein proximales Ende (21), das mit einem proximalen Anschluss (4) versehen ist, und ein distales Ende (22) umfasst, das dem proximalen Ende (21) entgegengesetzt ist, und
- einen distalen Teil (3), der einen distalen Durchgang (30) in Fluidverbindung mit dem proximalen Durchgang (20) definiert, wobei der distale Teil (3) fest mit dem distalen Ende (22) des proximalen Teils (2) verbunden ist,
wobei der distale Teil (3) aus einem Material besteht, das biegsamer ist als der proximale Teil (2), um seine Einführung in den Kanal zu erleichtern und jegliche Beschädigung des Kanals zu vermeiden,
wobei die Sonde (1) **dadurch gekennzeichnet ist, dass** der proximale Teil (2) einen Außendurchmesser zwischen 4/3 mm und 2,0 mm (zwischen 4 Fr und 6 Fr) aufweist, und dadurch, dass der distale Teil (3) mit dem proximalen Teil (2) einen Winkel (α) zwischen 30° und 60° bildet.

2. Sonde (1) nach Anspruch 1, wobei der proximale Teil (2) aus einem halbstarren Material gebildet ist, das eine Härte zwischen 60 Shore D und 100 Shore D, vorzugsweise zwischen 65 Shore D und 80 Shore D, zum Beispiel 72 Shore D, aufweist.

3. Sonde (1) nach einem der Ansprüche 1 oder 2, wobei der distale Teil (3) aus einem biegsamen Material gebildet ist, das eine Härte zwischen 20 Shore D und 60 Shore D, vorzugsweise zwischen 35 Shore D und 55 Shore D, aufweist.

4. Sonde (1) nach einem der Ansprüche 1 bis 3, wobei das Material des distalen Teils (3) und/oder des proximalen Teils (2) kalt von Hand plastisch verformbar ist.

5. Sonde (1) nach einem der Ansprüche 1 bis 4, wobei der proximale Teil ferner eine Öffnung (23) umfasst, die angepasst ist, um einen Versteifungsstab (24) aufzunehmen, der ausgestaltet ist, um den proximalen Teil (2) zu versteifen, zum Beispiel einen metallischen Stab.

6. Sonde (1) nach Anspruch 5, wobei der Versteifungsstab (24) gemeinsam mit der Öffnung (23) extrudiert oder in die Öffnung (23) eingeführt ist.

7. Sonde (1) nach einem der Ansprüche 1 bis 6, wobei der distale Teil (3) ein thermoplastisches Elastomermaterial vom Typ Polyether-Block-Amid (PEBA) umfasst.

8. Sonde (1) nach einem der Ansprüche 1 bis 7, wobei der distale Teil (3) mit dem proximalen Teil (2) einen Winkel (α) zwischen 40° und 45° bildet.

9. Sonde (1) nach einem der Ansprüche 1 bis 8, wobei der proximale Teil (2) und der distale Teil (3) gekrümmt sind.

10. Sonde (1) nach einem der Ansprüche 1 bis 9, wobei der proximale Teil (2) und/oder der distale Teil (3) eine Markierung (25, 35) umfasst.

11. Sonde (1) nach Anspruch 10, wobei der distale Teil (3) und der proximale Teil (2) eine unterschiedliche Farbe aufweisen.

12. Sonde (1) nach einem der Ansprüche 1 bis 11, wobei der distale Teil (3) in der Länge zwischen 0,5 cm und 4,0 cm misst, und der proximale Teil (3) in der Länge zwischen 15 cm und 25 cm misst.

## Claims

1. A probe (1) for injecting a surfactant into a trachea of a person, in particular a newborn or a premature baby, comprising:
- a proximal portion (2) defining a proximal passage (20), said proximal portion (2) comprising a proximal end (21) provided with a proximal connector (4) and a distal end (22), opposite to the proximal end (21), and
- a distal portion (3), defining a distal passage (30) in fluid communication with the proximal passage (20), said distal portion (3) being fixedly attached to the distal end (22) of the proximal portion (2),
the distal portion (3) being made of a more flexible material than the proximal portion (2) in order to facilitate its introduction into the duct and to avoid any damage to said duct,
the probe (1) being **characterized in that** the proximal portion (2) has an external diameter comprised between 4/3 mm and 2.0 mm (between 4 Fr and 6 Fr), and **in that** the distal portion (3) forms an angle (*α*) comprised between 30° and 60° with the proximal portion (2) .

2. The probe (1) according to claim 1, wherein the proximal portion (2) is formed in a semi-rigid material having a hardness comprised between 60 Shore D and 100 Shore D, preferably between 65 Shore D and 80 Shore D, for example 72 Shore D.

3. The probe (1) according to any of claims 1 or 2, wherein the distal portion (3) is formed in a flexible material having a hardness comprised between 20 Shore D and 60 Shore D, preferably between 35 Shore D and 55 Shore D.

4. The probe (1) according to any of claims 1 to 3, wherein the material of the distal portion (3) and/or the proximal portion (2) is plastically cold-deformable by hand.

5. The probe (1) according to any of claims 1 to 4, wherein the proximal portion (2) further comprises an opening (23), adapted to receive a stiffening rod (24) configured to stiffen said proximal portion (2), for example a metal rod.

6. The probe (1) according to claim 5, wherein the stiffening rod (24) is coextruded with the opening (23) or inserted into said opening (23).

7. The probe (1) according to any of claims 1 to 6, wherein the distal portion (3) comprises a thermoplastic elastomer material of the polyether block amide (PEBA) type.

8. The probe (1) according to any of claims 1 to 7, wherein the distal portion (3) forms an angle (*α*) comprised between 40° and 45° with the proximal portion (2) .

9. The probe (1) according to any of claims 1 to 8, wherein the proximal portion (2) and the distal portion (3) are curved.

10. The probe (1) according to any of claims 1 to 9, wherein the proximal portion (2) and/or the distal portion (3) comprises a marking (25, 35).

11. The probe (1) according to claim 10, wherein the distal portion (3) and the proximal portion (2) are in distinct colors.

12. The probe (1) according to any of claims 1 to 11, wherein the distal portion (3) measures between 0.5 cm and 4.0 cm long, and the proximal portion (3) measures between 15 cm and 25 cm long.
